# EUROPEAN PATENT APPLICATION

(11) **EP 4 108 177 A1**
(43) Date of publication of application: **28.12.2022**
(21) Application number: 21180499.2
(22) Date of filing: 21.06.2021
(51) Int. Cl.: A61B 8/00, G06T 7/00, G16H 30/40, G16H 40/60

(54) **INTRALUMINAL DEVICE DATA QUALITY METRIC**

(71) Applicant: Koninklijke Philips N.V., 5656 AG Eindhoven (NL)
(72) Inventor: GRASS, Michael, 5656 AE Eindhoven (NL); NICKISCH, Hannes, 5656 AE Eindhoven (NL); WISSEL, Tobias, 5656 AE Eindhoven (NL); SCHNELLBÄCHER, Nikolas David, 5656 AE Eindhoven (NL)
(74) Representative: Philips Intellectual Property & Standards

(57) **Abstract**

A system (100) for providing data quality metric values of an intraluminal device, is provided. The system includes one or more processors (120) configured to: receive (S120) intraluminal device data generated by an intraluminal device (110) at a plurality of positions along a lumen (140); display (S130) one or more extracorporeal images (130); and for at least some of the positions along the lumen (140), to: compute (S140) a value of a data quality metric (150) of the intraluminal device data at the position; determine (S150) a corresponding position of the intraluminal device (110) along the lumen (140) represented in the one or more displayed extracorporeal images (130); and indicate (S160) in the one or more displayed extracorporeal images (130), the value of the data quality metric (150) at the corresponding position.

## Description

### Technical Field

The present disclosure relates to providing a data quality metric for an intraluminal device. A system, a computer-implemented method, and a computer program product, are disclosed.

### Background

Minimally invasive procedures are often performed by navigating an intraluminal device within the anatomy under the guidance of an X-ray imaging system. Intraluminal devices often generate data during such procedures in order to provide an improved understanding of the anatomy. Imaging data such as intravascular ultrasound "IVUS" image data, and optical coherence tomography "OCT" image data is often generated during such procedures. Similarly, measurement data such as blood pressure measurement and blood flow data may also be generated during such procedures. X-ray image data generated by the X-ray imaging system provides context for the intraluminal device data. The X-ray image data and the intraluminal device data are often displayed together in order to facilitate a physician to make a diagnosis of the anatomy.

Various factors can, however, affect the quality of intraluminal device data, confounding its interpretation. In the example of IVUS image data, image artifacts such as guidewire artefacts, ring-down artefacts, reverberation artefacts, non-uniform rotational distortion "NURD", discontinuity artifacts, and catheter artefacts may occur. In the example of OCT, image artifacts may arise from incomplete lumen flushing, gas bubbles, saturation effects, tangential light drop-out, and sew-up effects. Similarly, measurement data such as blood pressure measurement and blood flow data may also include artifacts. In general, the origins of such artifacts may include intraluminal device motion, contact between the intraluminal device and the lumen wall, sub-optimal ECG gating, and mis-positioning of the intraluminal device.

As a result, physicians may take a nuanced approach to interpreting the data from intraluminal devices in order to ensure that their diagnosis is based on accurate data. This added burden can extend procedures, hampering workflow.

Consequently, there is a need for improvements to assist in the interpretation of intraluminal device data.

### Summary

According to one aspect of the present disclosure, a system for providing data quality metric values of an intraluminal device, is provided. The system includes one or more processors configured to:
- receive extracorporeal image data comprising one or more extracorporeal images representing a lumen;
- receive intraluminal device data generated by an intraluminal device at a plurality of positions along the lumen;
- display one or more of the extracorporeal images; and
- for at least some of the positions along the lumen:
   - compute a value of a data quality metric of the intraluminal device data at the position;
   - determine a corresponding position of the intraluminal device along the lumen represented in the one or more displayed extracorporeal images; and
   - indicate in the one or more displayed extracorporeal images, the value of the data quality metric at the corresponding position.

By indicating the value of the data quality metric in this manner, a physician may readily appreciate whether the data can be relied upon, alleviating some of the burden of its interpretation. Further aspects, features, and advantages of the present disclosure will become apparent from the following description of examples, which is made with reference to the accompanying drawings.

### Brief Description of the Drawings

Fig. 1 illustrates various examples of intraluminal device data in the form of IVUS images that include image artifacts: (A, B) originating from ostial lesions, (C) stems from a pullback through a heavily calcified bifurcation lesion in the right coronary artery, and (D) with the IVUS transducer within the catheter sheath.
Fig. 2 illustrates an example of intraluminal device data in the form of image longitudinal display (ILD) of IVUS imaging pullback data that includes image artifacts between markers A' - B and B' - C.
Fig. 3 illustrates an example of intraluminal device data in the form of intraluminal pressure measurements from a pressure wire: (A) proximal aortic pressure Pa, and (B) distal coronary pressure Pd.
Fig. 4 illustrates an example of intraluminal device data in the form of intraluminal pressure measurements from a pressure wire: (A) proximal aortic pressure Pa including artifacts arising from an unstable position of the guide catheter, and (B) distal coronary pressure Pd.
Fig. 5 illustrates an example of a system 100 for providing data quality metric values of an intraluminal device, in accordance with some aspects of the present disclosure.
Fig. 6 illustrates an example of a method for providing data quality metric values of an intraluminal device, in accordance with some aspects of the present disclosure.
Fig. 7 illustrates an example of a user interface including a displayed X-ray image 130 including a lumen 140, and an indication of values of a data quality metric 150 at positions along the lumen, in accordance with some aspects of the present disclosure.
Fig. 8 illustrates an example of a dashboard including a user's score for a procedure, in accordance with some aspects of the present disclosure.

### Detailed Description

Examples of the present disclosure are provided with reference to the following description and figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer implemented method, and in a computer program product, in a corresponding manner.

In the following description, reference is made to system in which data from an intraluminal device is generated from within the vasculature. Reference is made to an example of an intraluminal imaging device in the form of an IVUS imaging device. However, it is to be appreciated that the system may alternatively be used with other types of intraluminal devices, including intraluminal measurement devices that generate other types of data to image data. Examples of intraluminal imaging devices include an IVUS imaging device, an OCT device, an intracardiac echocardiography "ICE" device, and a transesophageal echocardiogram "TEE" probe. Examples of intraluminal measurement devices include a blood pressure measurement device, a blood flow measurement device, a position measurement device and their combination, e.g. combined pressure and flow velocity measurement device. Intraluminal measurement devices may include or more sensors, such as, and without limitation: a pressure sensor, a flow sensor, a temperature sensor, a force senor, an electrical conductance sensor, an electrical impedance sensor, an electrocardiogram sensor, a chemical sensor, an ultrasound sensor, and an optical sensor.

It is also to be appreciated that whilst the example IVUS imaging device referred-to herein is disposed within the vasculature, that other types of intraluminal devices may, as appropriate, be disposed within other types of lumens in the anatomy than the vasculature. The intraluminal device may for example be disposed within a lumen within the digestive tract, the colon, the esophagus, the bronchia and so forth.

It is also to be appreciated that the intraluminal device may be manipulated manually, or with the assistance of a robotic controller. In some instances, the intraluminal device is manipulated manually by the physician directly handling a portion of the intraluminal device as it exits the patient. In other instances the physician manually controls a robotic controller that provides a mechanical interface with the intraluminal device. In yet other instances, a robotic controller automatically manipulates the intraluminal device within the anatomy. Robotic controllers that use rollers and/or fingers that grip the intraluminal device in order to translate and/or rotate its distal end are known for this purpose.

Reference is made herein to generation of the extracorporeal imaging data, which is provided to the one or more processors of the system. The extracorporeal imaging data may be provided by: an X-ray based device in the form of X-ray images, a magnetic resonance imaging (MRI) system in the form of magnetic resonance images or an extracorporeal ultrasound system in the form of ultrasound images.

Reference is also made herein to the generation of X-ray image data. In this respect it is to be appreciated that the X-ray image data may be generated by various types of X-ray imaging systems. For example, the X-ray image data may be generated by a planar X-ray imaging system that generates planar X-ray images, or a volumetric X-ray imaging system that generates volumetric X-ray images. Planar X-ray imaging systems typically include a support arm such as a so-called "C-arm", or an "O-arm", that supports an X-ray source-detector arrangement. Planar X-ray imaging systems may alternatively include a support arm with a different shape to these examples. Planar X-ray imaging systems typically generate planar X-ray images with the support arm held in a static position with respect to an imaging region during the acquisition of image data. By contrast, volumetric X-ray imaging systems typically generate image data whilst rotating, or stepping, an X-ray source-detector arrangement around an imaging region, and subsequently reconstruct the image data obtained from multiple rotational angles into a volumetric image. Examples of volumetric X-ray imaging systems include computed tomography "CT" imaging systems, cone beam CT "CBCT" imaging systems, and spectral CT imaging systems.

Reference is further made to determination of a corresponding position of the intraluminal device along the lumen represented in the one or more displayed extracorporeal images, which can be performed by co-registration such as disclosed in US9375164B2 or EP2599033B1 entitled "Co-use of endoluminal data and extraluminal imaging", US10405828B2 entitled "Location determination apparatus", US8909322B2 entitled "Catheter for magnetic resonance guided procedures", US9808323B2 entitled "Visualization apparatus", incorporated by reference herein.

It is noted that the methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, the internet, or the cloud. The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD

As mentioned above, various factors can, affect the quality of intraluminal device data, leading to potential misinterpretation, e.g. confounding its content. In the example of IVUS image data, image artifacts such as guidewire artefacts, ring-down artefacts, reverberation artefacts, non-uniform rotational distortion "NURD", discontinuity artifacts, and catheter artefacts may occur. In the example of OCT image data, image artifacts may arise from incomplete lumen flushing, gas bubbles, saturation effects, tangential light drop-out, and sew-up effects. Similarly, measurement data such as blood pressure measurement and blood flow data from intraluminal measurement devices may also include artifacts. In general, the origins of such artifacts may include intraluminal device motion, contact between the intraluminal device and the lumen wall, sub-optimal ECG gating, and mis-positioning of the intraluminal device.

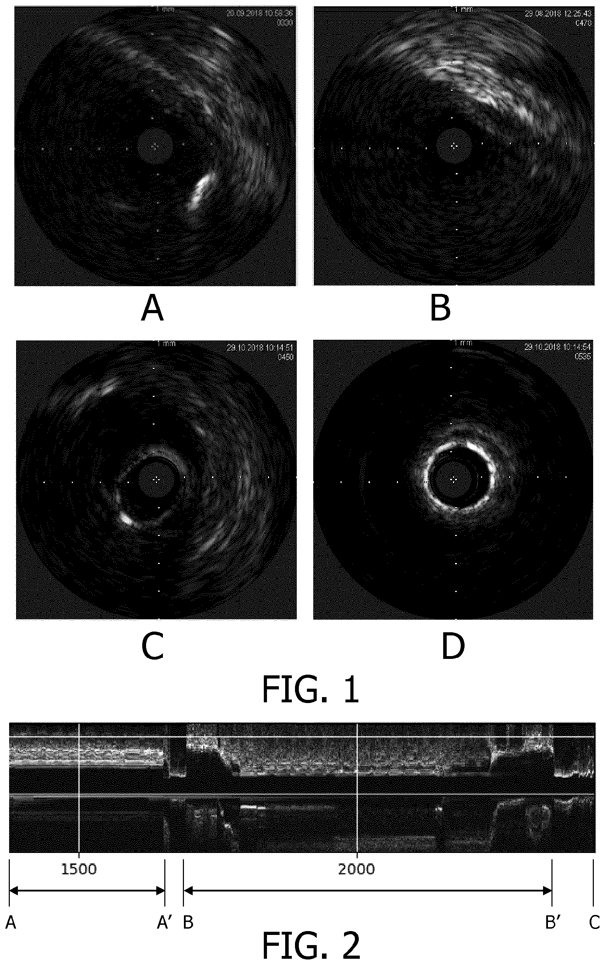

By way of some examples, Fig. 1 illustrates various examples of intraluminal device data in the form of IVUS images that include image artifacts: (A, B) originating from ostial lesions, (C) stems from a pullback through a heavily calcified bifurcation lesion in the right coronary artery, and (D) with the IVUS transducer within the catheter sheath. Fig. 1 (A, B) originate from ostial lesions where the cross-sectional field of view of the IVUS image, typically 10 millimeters, is exceeded by the vessel lumen and large portions of the relevant structures (arterial wall, intima etc.) lay outside the image. Fig. 1 (C) stems from a pullback through a heavily calcified bifurcation lesion in the right coronary artery where transducer jumps are likely to happen due to the rigidity of the vessel, and where acoustically dense materials lead to shadowing in the radial direction. In Fig. 1 (D) the IVUS transducer is within the catheter sheath, which is an acoustically dense layer that prevents the transducer from acquiring any information from the structures beyond it.

The image artifacts in the intraluminal data in Fig. 1 A - D are not only misleading but could also result in an erroneous diagnosis. For example, the missing data from the lower portion of the images in Fig. 1 (A) and (B), and the shadowing as well as branching in Fig. 1 (C) could result in inaccurate measurements of the lumen diameter. Structures that would radially bound the lumen contour are either not visible, or fall completely beyond the field of view. The presence of the catheter sheath in Fig. 1 (D) can also lead to an incorrect estimate of the lumen diameter because the bright ring-shaped appearance is ambiguous and can be misinterpreted e.g. as a stent or a calcium deposit. Such issues are particularly relevant when measurements are automated. The detection of such invalid intraluminal device data would therefore allow it to be excluded from quantitative assessment, and prevent erroneous diagnostic conclusions being reached based on such frames.

IVUS image data that is generated during a pullback procedure is often displayed in the form of a longitudinal section through the lumen, as illustrated in Fig. 2, which illustrates an example of intraluminal device data in the form of ILD of IVUS imaging pullback data that includes image artifacts between markers A' - B and B' - C. In contrast to the single images illustrated in Fig. 1, the longitudinal view of Fig. 2 provides additional context by illustrating the how the radial IVUS imaging signals vary along the lumen. As may be appreciated, abrupt changes in the apparent lumen diameter between the markers A' - B and B' - C in Fig. 2, also confound interpretation of such views. Such abrupt changes may provide an indicator to a physician not to make diagnostic decisions based on these artifact-affected regions of the lumen as they might be considered to represent image artifacts. However, this interpretation may also be inaccurate since this view represents only a cross section along the lumen. It may, for example, alternatively be the case that the apparently missing data in the displayed portion of the IVUS image data in Fig. 2 is actually the result of a bifurcation in the lumen, and therefore not caused by any artifact at all.

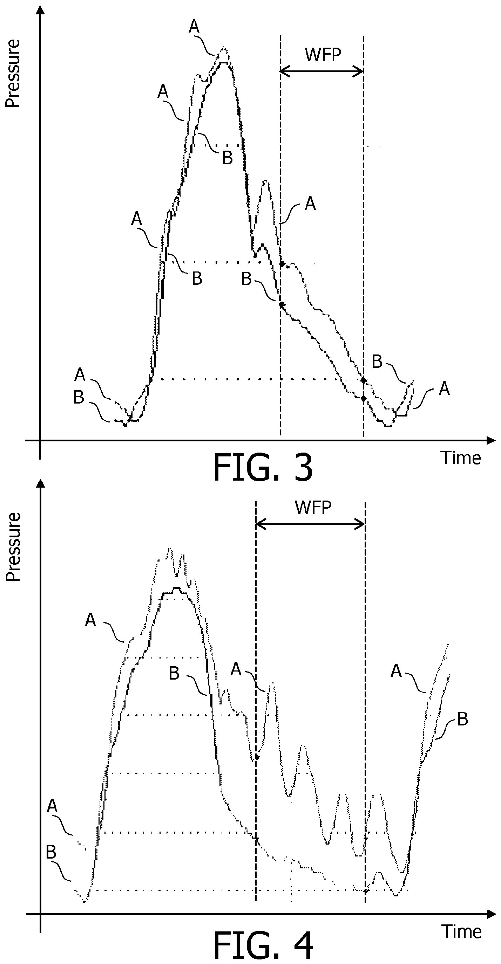

By way of another example, Fig. 3 illustrates an example of intraluminal device data in the form of intraluminal pressure measurements from a pressure wire: (A) proximal aortic pressure Pa, and (B) distal coronary pressure Pd. The traces A and B illustrated in Fig. 3 represent the pressure measured by the pressure wire over time, and are often acquired in order to make an instantaneous wave-free ratio "iFR" measurement. An iFR measurement may be used by a physician to diagnose whether a stenosis is causing a limitation of blood flow in coronary arteries. The iFR measurement is calculated as the ratio of the distal coronary pressure Pd to the proximal aortic pressure Pa, i.e. Pd/Pa, during the wave-free period labelled as WFP between the dashed lines in Fig. 3, and in which period the pressure is expected to be smoothly-varying. The traces in Fig. 3 vary as expected and are suitable for such a measurement. By contrast, Fig. 4 illustrates an example of intraluminal device data in the form of intraluminal pressure measurements from a pressure wire: (A) proximal aortic pressure Pa including artifacts arising from an unstable position of the guide catheter, and (B) distal coronary pressure Pd. In Fig. 4, in (A) there are signal artifacts in the form of oscillations in the expected wave free period WFP between the dashed lines in Fig. 3, and which could lead to an incorrect calculation of the iFR.

As a result of such issues, physicians may take a nuanced approach to interpreting the data from intraluminal devices in order to ensure that their diagnosis is based on accurate data. This added burden can extend procedures, hampering workflow.

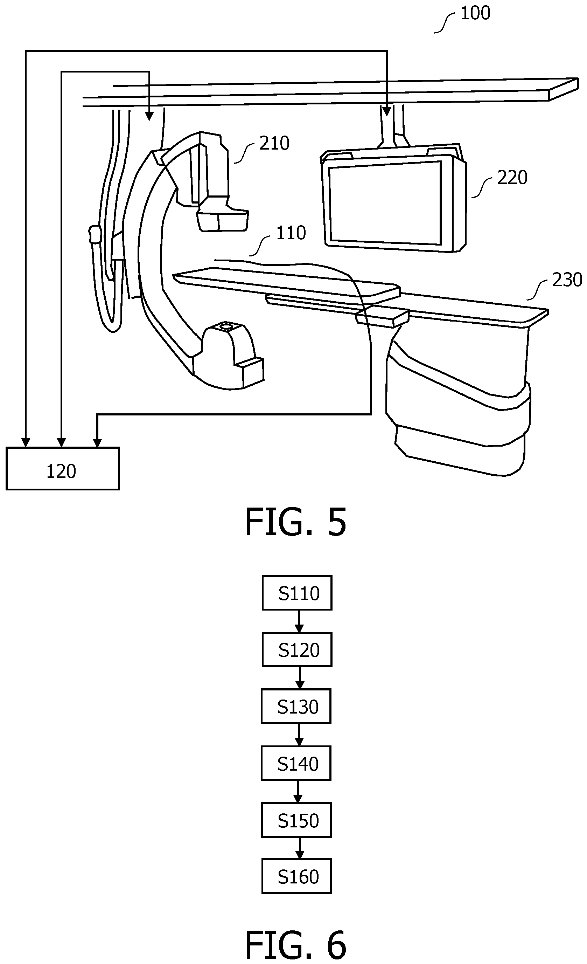

Fig. 5 illustrates an example of a system 100 for providing data quality metric values of an intraluminal device, in accordance with some aspects of the present disclosure. The system 100 includes one or more processors 120 configured to:
- receive S110 X-ray image data comprising one or more X-ray images 130 representing a lumen 140;
- receive S120 intraluminal device data generated by an intraluminal device 110 at a plurality of positions along the lumen 140;
- display S130 one or more of the X-ray images 130; and
- for at least some of the positions along the lumen 140:
   - compute S140 a value of a data quality metric 150 of the intraluminal device data at the position;
   - determine S150 a corresponding position of the intraluminal device 110 along the lumen 140 represented in the one or more displayed X-ray images 130; and
   - indicate S160 in the one or more displayed X-ray images 130, the value of the data quality metric 150 at the corresponding position.

Fig. 6 illustrates an example of a method for providing data quality metric values of an intraluminal device, in accordance with some aspects of the present disclosure. The operations performed in the method illustrated in Fig. 6 correspond to the operations mentioned above that are performed by the processor(s) 120 in the system 100. The method illustrated in Fig. 6 may be implemented by a computer. The method illustrated in Fig. 6 may also be provided in the form of instructions of a computer program product, or on a computer readable storage medium.

With reference to Fig. 5 and Fig. 6, in the operation S110, X-ray image data is received. The X-ray image data may be received via any form of data communication, including wired, optical, and wireless communication. By way of some examples, when wired or optical communication is used, the communication may take place via an electrical or optical cable, and when wireless communication is used, the communication may for example be via RF or infrared signals. The X-ray image data may be received from an X-ray imaging system 210 as illustrated in Fig. 5. Various types of X-ray imaging system may be used to generate the X-ray image data, as mentioned above. The X-ray image data may alternatively be received from another source, such as a for example a computer readable storage medium, the internet, the cloud, and so forth.

The received X-ray image data includes one or more X-ray images 130 representing a lumen 140. In general, the one or more X-ray images 130 may include a single X-ray image, or a temporal sequence of X-ray images. If provided as a temporal sequence, the temporal sequence of X-ray images may be live images in the sense that the temporal sequence of X-ray images are generated instantaneously before being received in the operation S110. Alternatively, the temporal sequence may have been generated some seconds, minutes, hours, or even days, or a longer period, beforehand. The lumen 140 represented in the X-ray images 130 may be a lumen within the vasculature for example, or indeed another lumen within the anatomy.

In the operation S120, intraluminal device data generated by an intraluminal device 110 at a plurality of positions along the lumen 140, is received. In general, the intraluminal device may be an intraluminal imaging device, or an intraluminal measurement device. The intraluminal device data may therefore include image data, or other types of measurement data such as blood pressure measurement data. The intraluminal device data may, as described for the X-ray image data, be received via any form of data communication.

As illustrated in Fig. 5, the intraluminal device data may be received from an intraluminal device 110, such as for example an IVUS imaging device, or indeed another type of intraluminal device. The intraluminal device data may be live data in the sense that the intraluminal device data is generated instantaneously before being received in the operation S120. The intraluminal device data may alternatively be received from another source, such as for example a computer readable storage medium, the internet, the cloud, and so forth. The intraluminal device data may therefore have been generated some seconds, minutes, hours, or even days, or a longer period, beforehand.

The intraluminal device data may be generated at a plurality of positions along the lumen 140 by, for example, translating the intraluminal device along the lumen and generating data at different axial positions along the length of the lumen. In the example of the intraluminal device being an IVUS imaging device, the intraluminal device data may be generated during a so-called pullback procedure. The pullback procedure may be performed manually, or using a robotic controller. The intraluminal device data may be generated periodically, for example at regular time steps corresponding to the image frames of an intraluminal imaging device, or aperiodically.

With continued reference to Fig. 5 and Fig. 6, in the operation S130, the one or more of the X-ray images 130 is displayed. The image(s) may for example be displayed on a display such as monitor 220 illustrated in Fig. 5, or on another display such as the display of a console, desktop PC, or a tablet, for example.

In the operation S140, for at least some of the positions along the lumen 140, the value of a data quality metric 150 of the intraluminal device data at the position, is computed. The positions along the lumen at which the value of the data quality metric 150 is computed, may be each position at which the intraluminal device data is generated, or a subset of these positions. For example, the value of the data quality metric may computed at every n^{th} position, where n represents an integer. Alternatively, the value of the data quality metric may be computed at regular spatial intervals along the length of the lumen.

Various data quality metrics may be computed in the operation S140. In examples wherein the intraluminal device is an intraluminal imaging device, the intraluminal device data may include image data and the data quality metric may include an image quality metric of the image data. The image quality metric may for example be computed based on the presence of one or more in the following in the image data: a guidewire artifact, a ring-down artifact, a reverberation artifact, a non-uniform rotational distortion artifact, a discontinuity artifact, a catheter artifact, an artifact due to incomplete lumen flushing, a gas bubble artifact, a saturation artifact, a tangential light drop-out artifact, a sew-up artifact, a motion artifact and an electrocardiographic gating artifact. The image quality metric may also be computed based on the presence of other types of artifacts in the image data, including noise.

Some types of intraluminal device data, for example IVUS and OCT image data, include an angular variation around an axis of the lumen 140. In such cases it may be beneficial to determine the angular variation of the data quality metric, or a composite value for the angular variation of the data quality metric. This may be used to alert a physician to positions in which only a portion of the data at the position, is affected by an artifact, potentially allowing the remainder of the data at the position to be used for diagnostic purposes. Thus, in one example, the intraluminal device data, and the corresponding computed values of the data quality metric, comprise an angular variation around an axis of the lumen 140 at the position; and the one or more processors are configured to indicate in the one or more displayed X-ray images 130, the value of the data quality metric at the corresponding position, by:
- displaying a graphical representation of: the angular variation of the data quality metric at the position and/or the angular variation of the intraluminal device data at the position; or
- displaying a graphical representation of: a composite value representing the angular variation of the data quality metric at the position and/or a composite value representing the angular variation of the intraluminal device data at the position.

Suitable graphical representations of the angular variation include for example displaying the data quality metric on a polar plot, or by projecting the data quality metric onto the inner surface of a cylinder and un-rolling the cylinder to provide a planar view. The composite value may be determined by, for example, computing the average values at the position, or a weighted average thereof. These may be displayed numerically, or by way of an intensity or a colour indicating the value, for example.

In examples wherein the intraluminal device is an intraluminal measurement device, data quality metrics representing data artifacts such as a signal-to-noise ratio, a change in signal bias, signal shift, and signal spikes, and also more complex data quality metrics, may be computed, as described in the examples below.

In one example, the value of the data quality metric of the intraluminal device data is computed by applying a feature detection algorithm to the intraluminal device data. In this example, a modality-specific and artifact-specific feature detector is applied to the intraluminal device data and used to label the data if the relevant artifact is detected. The detection of specific artifacts may be detected by detecting characteristics of each artifact type. For example, ring-down or reverberation artifacts in IVUS images may be detected by performing an image-based match with a template of approximately concentric rings at predetermined radial distances from the IVUS transducer. Ring-down artefacts typically occur closer to the transducer, and reverberation further out from the transducer. The IVUS image, in polar or cartesian representation, may also be transformed into the spectral domain in order to detect artifact-specific characteristics. Reverberation artefacts occur repetitively in a direction radially outwards from the IVUS transducer. NURD artefacts exhibit low spatial variation with polar angle. In the case of NURD artifacts, a sliding-window spectral analysis or wavelet analysis may be performed in order to detect as these artifacts since they are often confined to subregions of the IVUS image. Catheter artifacts such as illustrated in Fig. 1 (D), and arise from the IVUS transducer's emitted ultrasound signals being obscured by the catheter or its sheath, may be detected via the absence of IVUS signal around a significant portion of the polar angle at radial distances that are close to the transducer. Discontinuities may also be detected as abnormal peaks in the spectral domain, e.g. as higher frequencies along the polar angle dimension due to a signal jump, or as texture discontinuities in the IVUS image via e.g. spatial high-pass filtering.

In another example, the value of the data quality metric of the intraluminal device data is computed by inputting the intraluminal device data into a neural network trained to detect data artifacts. In this example, a regression-type neural network is trained to detect artifacts using a supervised learning process based on training data that includes image and/or other measurement data artifacts. Prior to inputting the intraluminal device data into the neural network, at inference, the intraluminal device data may be pre-processed in order to emphasize the artifacts. This may for example include transforming the intraluminal device data into the spectral domain, or performing a sliding-window spectral analysis or wavelet analysis. At inference, the neural network may output an analogue value representing the severity of the data quality metric. The analogue value may also be digitalized by applying a threshold in order to classify the data as being affected by a data artifact, or otherwise free from data artifacts. After performing inference, the inputted intraluminal device data, may therefore be labelled with the result of the analysis. For example, an inputted IVUS image may be labelled, or a region within the IVUS image may be labelled with an identifier to indicate the presence of an artifact if the output of the neural network exceeds a predetermined threshold. In a similar manner, a neural network may be trained, and subsequently used during inference, to perform a regression analysis on inputted data signals representing other types of intraluminal device data such as the intraluminal pressure measurements in Fig. 3, or intraluminal flow measurements, and so forth.

In another example, the value of the data quality metric of the intraluminal device data is computed by inputting the intraluminal device data into a neural network trained to classify the inputted intraluminal device data as belonging to one or more expected classifications, and identifying outlier data not belonging to the one or more expected classifications as artifact data. In this example, a classification-type neural network may be used to perform an image classification or segmentation task on the inputted intraluminal device data if this represents image data. In the example of IVUS image data, image regions representing the lumen, plaque, or a stent, may be classified. Techniques such as out-of-distribution "OOD" detection may be used to provide a value representing how different an inputted image is from a high quality, i.e. artifact-free, image. In an example OOD-method, the weight distribution of the inferred network may also be analyzed to detect abnormal images which do not belong to a training dataset of high quality IVUS images. In a similar manner, a neural network may be trained, and subsequently used during inference to classify inputted data signals representing other types of intraluminal device data such as the intraluminal pressure measurements in Fig. 3, or intraluminal flow measurements, and so forth.

A combination of these techniques may also be used to generate a data quality metric, for example by combining their values as a weighted sum.

In one example, the data quality metric for each position along the lumen 140 is computed based on the intraluminal device data at the position along the lumen, as well as the intraluminal device data at one or more neighbouring positions. The additional information that is provided by the data at the neighbouring position(s) may provide additional context that improves the accuracy of the computed data quality metric. For example, if a bifurcation is detected in IVUS images, an abrupt change in lumen diameter in the IVUS images might be mis-interpreted as an artifact if only a single IVUS image is analysed. However, by also analysing one or more neighbouring IVUS images, the additional data from these neighbouring images may be used to assign the appropriate data quality metric based on the information that the image is in the vicinity of the bifurcation. In this example, the one or more processors are configured to compute the value of the data quality metric of the intraluminal device data at the position based on the intraluminal device data at the position, and on the intraluminal device data at one or more neighbouring positions.

Returning to Fig. 6, in the operation S150, a corresponding position of the intraluminal device 110 along the lumen 140 represented in the one or more displayed X-ray images 130, is determined. In this operation, the position of a portion of the intraluminal device may be detected, such as the location of an intraluminal device data-generating transducer disposed thereon, or its distal end.

In some examples, image-based techniques are used to detect the position of the intraluminal device 110 in the X-ray image(s) 130 in the operation S150. In one image-based approach, the position of the intraluminal device 110 in the X-ray image(s) 130 is detected using a feature detection algorithm. The feature detection algorithm may identify the location in the X-ray image(s) of a portion of the intraluminal device by representing the local image appearance with descriptors such as SURF, SIFT, or ORB. Subsequently, those descriptors are quantized using e.g. k-mean clustering. The histogram of the descriptor cluster matches are used as features for a learning machine such as a support vector machine "SVM" to finally detect the portion of the intraluminal device. In another image-based technique, the position of the intraluminal device 110 in the X-ray image(s) 130 is detected using a neural network such as a convolutional neural network. A supervised learning process may be used to train the neural network using training dataset of X-ray images including the intraluminal device, and which are labelled with the location of a portion of the interventional device.

In other examples, tracking data generated by a tracking system is used to determine a position of the intraluminal device in the one or more X-ray images. In this respect, tracking data may be generated by various types of tracking systems, including electromagnetic "EM" tracking systems such as the Carto^{®} system marketed by Biosense Webster, USA, and so-called optical shape sensing systems that employ one or more optical fibers with strain sensors to detect the position of an intraluminal device, as disclosed in document WO 2012/101563 A2. Thus, in these examples, the one or more processors determine the corresponding position of the intraluminal device along the lumen represented in the one or more displayed X-ray images 130 based on tracking data representing a position of the intraluminal device in the one or more X-ray images 130.

Tracking systems typically detect the location of a tracked element, such as an (electro)magnetic device, or an optical fiber, that is coupled to the intraluminal device, in a coordinate system of the tracking system. The location of the tracked element is then mapped into a coordinate system of the X-ray imaging system, and thus to a location in the X-ray image(s) using a mapping function that is determined by registering the coordinate systems to one another. The coordinate systems may be registered to one another based on the detection of the locations of reference locations that are common to both the X-ray imaging system and the tracking system.

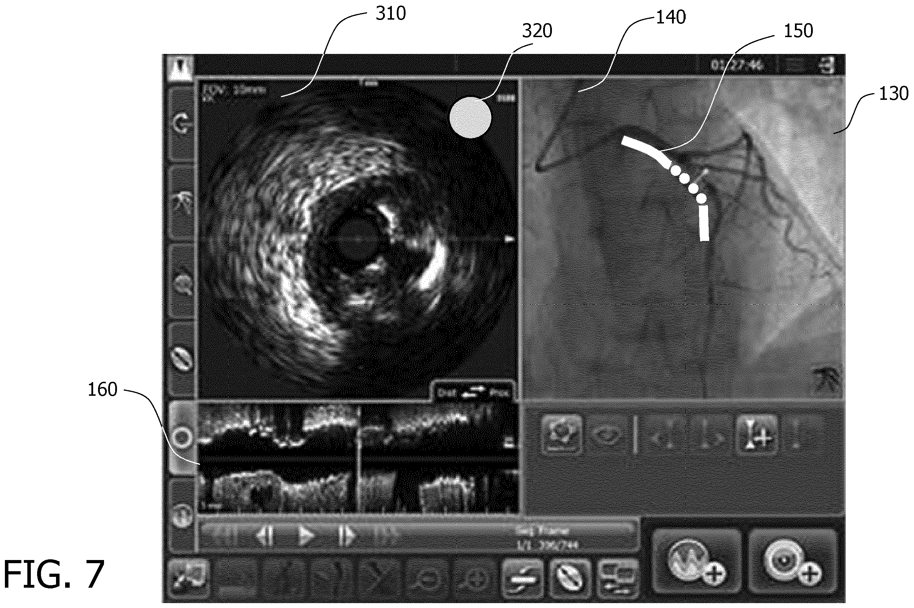

Returning to Fig. 6, in the operation S160, the value of the data quality metric 150 is indicated in the displayed X-ray image(s) 130, at the corresponding position. The data quality metric may for example be indicated as an overlay. Thus in one example, the value of the data quality metric is indicated at the corresponding position, by overlaying the one or more displayed X-ray images 130 with a graphical representation of the value of the data quality metric at the corresponding position. The graphical representation may for example include a trace that is displayed on top of or adjacent to the lumen 140, and with a colour that is indicative of the value of the data quality metric. A high value of the data quality metric may for example be represented by light contrast or a green colour, whereas a low value may be represented in darker contrast or in a red colour. In another example, a high value of the data quality metric may be represented by a continuous trace in light contrast, whereas a low value may be represented as a dashed trace in light contrast, and with intermediate values being represented via the length of the dashes in the trace. This is shown in Fig. 7, which illustrates an example of a user interface including a displayed X-ray image 130 including a lumen 140, and an indication of values of a data quality metric 150 at positions along the lumen, in accordance with some aspects of the present disclosure.

With reference to Fig. 7, in this example, the displayed X-ray image 130 is a reference image that is generated prior to the intraluminal device being translated along the lumen 140. In Fig. 7, the value of the data quality metric 150 is indicated as being high along the sections of the lumen 140 corresponding to the continuous trace in light contrast, and low in the curved section of the lumen 140 corresponding to the dashed trace in light contrast.

By displaying the value of the data quality metric 150 in this manner, a physician may quickly determine regions along the lumen 140 displayed X-ray image 130 at which the data is of sufficiently high quality for diagnostic purposes. It therefore alleviates some of the burden of interpreting the data, allowing the physician to focus on a diagnosis, and other aspects of the procedure. The physician may also use the data quality metric for other purposes. For example, they may use it to identify sections of the lumen along which the data acquisition should be repeated in order to provide more reliable data. Providing the feedback in this manner also gives the physician the opportunity to adjust the manner in which the intraluminal device is translated along the lumen, for example to adjust its rate, in order to obtain more reliable intraluminal device data in future, which also improves workflow.

The user interface illustrated in Fig. 7 may be in communication with a user input device to facilitate a physician to execute various operations described above. For example, it may include a keyboard, a touch screen, or a pointer device such as a mouse to measure the lumen diameter, and so forth.

In the example illustrated in Fig. 7, the displayed X-ray image 130 is a static reference image that is generated prior to the intraluminal device being translated along the lumen 140. Displaying a static X-ray image such as that in Fig. 7 permits a straightforward interpretation of the position of the intraluminal device since the displayed lumen in the X-ray image does not move.

In general, in the operation S130, it is contemplated to display a pre-procedural image, or an intra-procedural image or a post-procedural image, and in the operation S160 it is contemplated to indicate the value of the data quality metric 150 at the corresponding position in the displayed X-ray image 130. The X-ray image 130 may also be updated intermittently. In some examples, the pre-procedural, or intra-procedural, or post-procedural X-ray image may be segmented in order to identify a portion of the coronary vasculature. In one example, the processor(s) 120 may also receive a tomographic image representing the lumen. In this example, the processor(s) 120 may display the tomographic image and register the displayed X-ray image(s) to the tomographic image. The additional context provided by the tomographic image may facilitate the physician in navigating the intraluminal device, or in performing a diagnosis on the lumen 140. It is also contemplated to generate a temporal sequence of X-ray images, and to select an X-ray image from the temporal sequence in which to indicate the value of the data quality metric 150. The displayed X-ray image 130 may be a live, i.e. a current X-ray image from a temporal sequence of X-ray images, or an image from an earlier point in time, and which is used as a static reference image as described above.

In one example, a temporal sequence of X-ray images is generated, and image-based techniques are used to detect the position of the intraluminal device 110 in the X-ray images in the operation S150. In this example, the X-ray image data comprises a temporal sequence of X-ray images, and the intraluminal device data is generated contemporaneously with the temporal sequence of X-ray images. The one or more processors are configured to determine the corresponding position of the intraluminal device along the lumen represented in the one or more displayed X-ray images 130, by analysing the temporal sequence of X-ray images to identify the positions of the intraluminal device in the X-ray images. The one or more processors are configured to display the one or more of the X-ray images 130 by displaying a selected one of the X-ray images in the temporal sequence.

In this example, the selected X-ray image may be any of the X-ray images in the temporal sequence. For example, it may be an intra-procedural image, such as a live, i.e. a current, X-ray image in the sequence, and in which case the image is updated repeatedly. Alternatively, the selected X-ray image may be an X-ray image that is generated at an earlier point in time to the current time. If provided by an intra-procedural image, the intra-procedural image may be updated intermittently, as described above. In one variant of this approach, rather than displaying the one or more of the X-ray images 130 by displaying a selected one of the X-ray images in the temporal sequence; in an alternative implementation, a pre-procedural X-ray image may be generated prior to the generation of the intraluminal device data, and the position displayed in this image, whilst the corresponding position of the intraluminal device along the lumen represented in the one or more displayed X-ray images 130, is likewise determined by analysing the temporal sequence of X-ray images to identify the positions of the intraluminal device in the X-ray images. In this example, the X-ray image data further comprises a pre-procedural X-ray image generated prior to the generation of the intraluminal device data, and the one or more processors are configured to display the one or more of the X-ray images by displaying the pre-procedural X-ray image.

In one example, a graphical representation 160 of the intraluminal device data is also displayed. In this example, the one or more processors 120 are also configured to display a graphical representation 160 of the intraluminal device data. The graphical representation includes the data generated by the intraluminal device 110 at the plurality of positions along the lumen 140. An example of this is illustrated in Fig. 7, and wherein a cross sectional view of intraluminal device data from an IVUS imaging device from a pullback procedure, is illustrated. Displaying the intraluminal device data may further facilitate a physician to make a diagnosis of the lumen. One or more markers may also be provided in the displayed graphical representation 160 to indicate the value of the data quality metric along one or more portions of the lumen 140.

The graphical representation 160 may additionally or alternatively include a graphical representation 310 of the intraluminal device data from a current position of the interventional device along the lumen. In Fig. 7, this is illustrated for example IVUS image data that is generated in a radial direction with respect to the axis of the lumen 140. In Fig. 7, the value of the data quality metric for the intraluminal device data from the current position of the interventional device along the lumen 320, is also displayed. In Fig. 7, the value of the data quality metric is indicated by means of a coloured shape 320 that changes colour in accordance with the value of the data quality metric. The value of the data quality metric may alternatively be indicated via an intensity or saturation of a colour, or by displaying the value numerically, or as an icon. The icon may for example change in response to the value of the data quality metric in order to represent high, medium, and low values. Providing the value of the data quality metric for the current position of the interventional device in this manner helps to indicate to a physician whether the current position of the interventional device is a suitable position to make a measurement of the lumen diameter, for example.

Having obtained intraluminal device data for positions along the lumen 140, it may be the case that the data quality metric is inadequate for diagnostic purposes in a critical section of the lumen. For example, there may be IVUS image data artifacts at positions within a plaque, and the physician desires an accurate measurement of the lumen diameter at that position. In one example, the physician may then use the intraluminal device to generate a second set of intraluminal device data along the lumen, i.e. second intraluminal device data. The system 100 computes values of the data quality metric for the second intraluminal device data, and replaces the intraluminal device data in the displayed graphical representation 160, with the second intraluminal device data at the positions where artifacts are detected if the second intraluminal device data is of higher quality.

In this example, the one or more processors 120 are further configured to display a graphical representation 160 of the intraluminal device data. The one or more processors also:
- receive second intraluminal device data generated by the intraluminal device at the plurality of positions along the lumen 140; and
- for at least some of the positions along the lumen 140:
   compute a value of the data quality metric of the second intraluminal device data at the position;
   determine a corresponding position of the intraluminal device along the lumen 140 represented in the one or more displayed X-ray images 130; and
   replace the intraluminal device data in the displayed graphical representation 160 of the intraluminal device data at the corresponding position, with the second intraluminal device data if the value of the data quality metric of the second intraluminal device data at the position is higher than the value of the data quality metric of the intraluminal device data at the corresponding position; such that the higher value of the data quality metric is indicated in the one or more displayed X-ray images 130 at the position in which the intraluminal device data is replaced.

In so doing, more accurate intraluminal device data may be provided for the lumen 140, thereby facilitating the physician to provide a more informed diagnosis on the lumen.

In this example, the one or more processors 120 may be configured to replace the intraluminal device data automatically, or contingent on receiving user input confirming that the intraluminal device data shall be replaced. For example, the system may display a prompt on the user interface illustrated in Fig. 7 requesting the physician to confirm that the intraluminal device data along the dashed section 150 should be replaced by higher quality data from a further IVUS pullback operation. In so doing, the physician is provided with the option to over-ride the replacement of the data.

In one example, the computed values of the data quality metric at the positions along the lumen are combined into a score. A score is computed for a procedure, for example for a pullback procedure. The score may then be used for various purposes. For example, it may be displayed in order to give feedback to the physician in order to guide their decision on whether or not to repeat the procedure to obtain data with less artifacts, or to guide the physician to improve their technique. The score may be used to label the intraluminal device data for future reference, or to determine which of multiple sets of data from different procedures to store to memory. Thus, in one example, the one or more processors 120 are configured to receive the X-ray image data, and to receive the intraluminal device data, and to display the one or more of the X-ray images 130, and to compute the value of a data quality metric, and to determine the corresponding position of the intraluminal device, and to indicate in the one or more displayed X-ray images 130, the value of the data quality metric at the corresponding position, for a plurality of procedures. In this example, the one or more processors 120 are further configured to:
- compute a score 170 for each procedure based on the computed values of the data quality metric in the procedure; and
- display the score for one or more of the procedures; and/or
- label the intraluminal device data for the procedure with the score; and/or
- store to a computer readable storage medium, only the intraluminal device data for the procedure with the highest score.

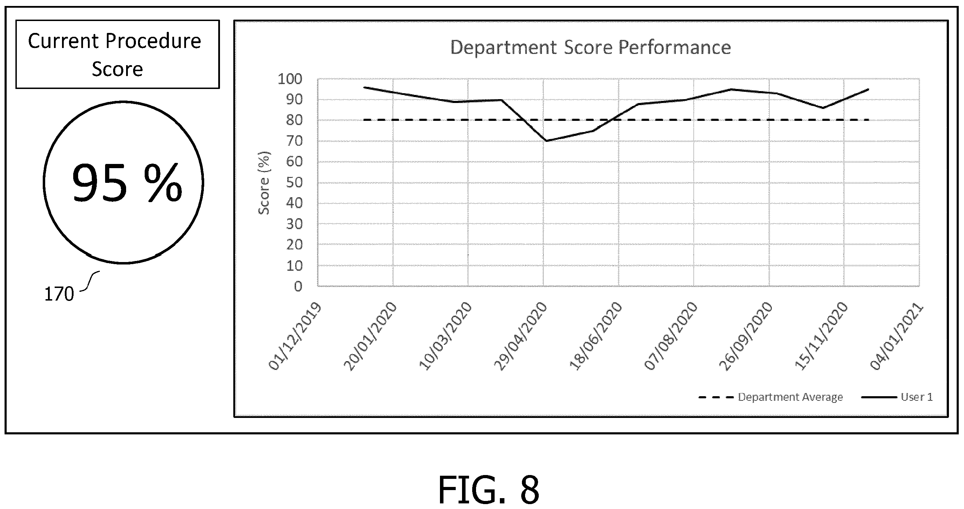

This example is illustrated with reference to Fig. 8, which illustrates an example of a dashboard including a user's score for a procedure, in accordance with some aspects of the present disclosure. As illustrated in Fig. 8, the score 170 for the current procedure is displayed via the dashboard. Moreover, the user's performance over time may also be displayed on the dashboard, together with the average of the scores for one or more other users, such as a group of users in a department of a medical facility. By providing feedback to a user in this manner, the physician may be guided to improve their performance over time, resulting in more efficient workflow.

The system 100 described above may also include one or more of: an X-ray imaging system 210, a monitor 220, and a patient bed 230, as illustrated in Fig. 5. The system may also include a user input device such as a keyboard, and/or a touch screen, and/or a pointing device such as a mouse (not illustrated in Fig. 1) for executing the various operations. The system elements are in communication with each other as illustrated by way of the interconnecting lines in Fig. 1

Aspects of the above-described system 100 may, as mentioned above, also be provided in the form of a computer implemented method. Thus, a computer-implemented method for providing data quality metric values of an intraluminal device includes:
- receiving S110 X-ray image data comprising one or more X-ray images 130 representing a lumen 140;
- receiving S120 intraluminal device data generated by an intraluminal device 110 at a plurality of positions along the lumen 140;
- displaying S130 one or more of the X-ray images 130; and
- for at least some of the positions along the lumen 140:
   computing S140 a value of a data quality metric 150 of the intraluminal device data at the position;
   determining S150 a corresponding position of the intraluminal device 110 along the lumen 140 represented in the one or more displayed X-ray images 130; and
   indicating S160 in the one or more displayed X-ray images 130, the value of the data quality metric 150 at the corresponding position.

The computer implemented method may also include one or more further operations described above in relation to the system 100.

Aspects of the above-described system 100 may, as mentioned above, also be provided in the form of a computer program product. The computer program product comprises instructions which when executed by one or more processors, cause the one or more processors to carry out the aforementioned computer implemented method. The computer program product may also include instructions to carry out one or more further operations described above in relation to the system 100.

The above examples are to be understood as illustrative of the present disclosure, and not restrictive. Further examples are also contemplated. For instance, the examples described in relation to the system 100, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for providing data quality metric values of an intraluminal device, the system comprising one or more processors (120) configured to:
receive (S110) extracorporeal image data comprising one or more extracorporeal images (130) representing a lumen (140);
receive (S120) intraluminal device data generated by an intraluminal device (110) at a plurality of positions along the lumen (140);
display (S130) one or more of the extracorporeal images (130); and
for at least some of the positions along the lumen (140):
compute (S140) a value of a data quality metric (150) of the intraluminal device data at the position;
determine (S150) a corresponding position of the intraluminal device (110) along the lumen (140) represented in the one or more displayed extracorporeal images (130); and
indicate (S160) in the one or more displayed extracorporeal images (130), the value of the data quality metric (150) at the corresponding position.

2. The system according to claim 1, wherein the extracorporeal image data comprises:
a pre-procedural extracorporeal image generated prior to the generation of the intraluminal device data, and wherein the one or more processors (120) are configured to display the one or more of the extracorporeal images (130) by displaying the pre-procedural extracorporeal image; or
an intra-procedural extracorporeal image generated prior to the generation of the intraluminal device data, and wherein the one or more processors (120) are configured to display the one or more of the extracorporeal images (130) by displaying the intra-procedural extracorporeal image; or
a post-procedural extracorporeal image generated after the generation of the intraluminal device data, and wherein the one or more processors (120) are configured to display the one or more of the extracorporeal images (130) by displaying the post-procedural extracorporeal image; or
a temporal sequence of extracorporeal images, and wherein the one or more processors (120) are configured to display the one or more of the extracorporeal images (130) by displaying a selected one of the extracorporeal images from the temporal sequence.

3. The system according to claim 1, wherein the one or more processors (120) are configured to compute the value of the data quality metric of the intraluminal device data at the position, by:
applying a feature detection algorithm to the intraluminal device data; and/or
inputting the intraluminal device data into a neural network trained to detect data artifacts; and/or
inputting the intraluminal device data into a neural network trained to classify the inputted intraluminal device data as belonging to one or more expected classifications, and identifying outlier data not belonging to the one or more expected classifications as artifact data.

4. The system according to claim 1, wherein the one or more processors (120) are configured to compute the value of the data quality metric of the intraluminal device data at the position based on the intraluminal device data at the position, and on the intraluminal device data at one or more neighbouring positions.

5. The system according to claim 1, wherein the extracorporeal image data comprises a temporal sequence of extracorporeal images;
wherein the intraluminal device data is generated contemporaneously with the temporal sequence of extracorporeal images;
wherein the one or more processors (120) are configured to determine the corresponding position of the intraluminal device along the lumen (140) represented in the one or more displayed extracorporeal images, by analysing the temporal sequence of extracorporeal images to identify the positions of the intraluminal device in the extracorporeal images; and
wherein the one or more processors (120) are configured to display the one or more of the extracorporeal images by displaying a selected one of the extracorporeal images in the temporal sequence; or
wherein the extracorporeal image data further comprises a pre-procedural extracorporeal image generated prior to the generation of the intraluminal device data, and wherein the one or more processors (120) are configured to display the one or more of the extracorporeal images by displaying the pre-procedural extracorporeal image.

6. The system according to claim 1, wherein the one or more processors (120) are configured to determine the corresponding position of the intraluminal device along the lumen (140) represented in the one or more displayed extracorporeal images based on tracking data representing a position of the intraluminal device in the one or more extracorporeal images (130).

7. The system according to claim 1, wherein the one or more processors (120) are further configured to display a graphical representation (160) of the intraluminal device data; and wherein the one or more processors (120) are further configured to:
receive second intraluminal device data generated by the intraluminal device at the plurality of positions along the lumen (140); and
for at least some of the positions along the lumen (140):
compute a value of the data quality metric of the second intraluminal device data at the position;
determine a corresponding position of the intraluminal device along the lumen (140) represented in the one or more displayed extracorporeal images (130); and
replace the intraluminal device data in the displayed graphical representation (160) of the intraluminal device data at the corresponding position, with the second intraluminal device data if the value of the data quality metric of the second intraluminal device data at the position is higher than the value of the data quality metric of the intraluminal device data at the corresponding position; such that the higher value of the data quality metric is indicated in the one or more displayed extracorporeal images (130) at the position in which the intraluminal device data is replaced.

8. The system according to claim 7, wherein the one or more processors (120) are configured to replace the intraluminal device data automatically, or contingent on receiving user input confirming that the intraluminal device data shall be replaced.

9. The system according to claim 1, wherein the one or more processors (120) are configured to indicate in the one or more displayed extracorporeal images (130), the value of the data quality metric at the corresponding position, by overlaying the one or more displayed extracorporeal images (130) with a graphical representation of the value of the data quality metric at the corresponding position.

10. The system according to claim 1, wherein the intraluminal device data, and the corresponding computed values of the data quality metric, comprise an angular variation around an axis of the lumen (140) at the position; and
wherein the one or more processors (120) are configured to indicate in the one or more displayed extracorporeal images (130), the value of the data quality metric at the corresponding position, by:
displaying a graphical representation of: the angular variation of the data quality metric at the position and/or the angular variation of the intraluminal device data at the position; or
displaying a graphical representation of: a composite value representing the angular variation of the data quality metric at the position and/or a composite value representing the angular variation of the intraluminal device data at the position.

11. The system according to any previous claim, wherein the one or more processors (120) are configured to display a graphical representation of the intraluminal device data.

12. The system according to any previous claim, wherein the intraluminal device comprises an intraluminal imaging device or an intraluminal measurement device, and/or wherein the extracorporeal imaging data is provided by an X-ray device.

13. The system according to claim 12, wherein the intraluminal device comprises an intraluminal imaging device, wherein the intraluminal device data comprises image data and wherein the data quality metric comprises an image quality metric of the image data.

14. The system according to claim 13, wherein the image quality metric is computed based on the presence of one or more in the following in the image data: a guidewire artifact, a ring-down artifact, a reverberation artifact, a non-uniform rotational distortion artifact, a discontinuity artifact, a catheter artifact, an artifact due to incomplete lumen flushing, a gas bubble artifact, a saturation artifact, a tangential light drop-out artifact, a sew-up artifact, a motion artifact and an electrocardiographic gating artifact.

15. The system according to any previous claim, wherein the one or more processors (120) are configured to receive the extracorporeal image data, and to receive the intraluminal device data, and to display the one or more of the extracorporeal images (130), and to compute the value of a data quality metric, and to determine the corresponding position of the intraluminal device, and to indicate in the one or more displayed extracorporeal images (130), the value of the data quality metric at the corresponding position, for a plurality of procedures; and
wherein the one or more processors (120) are further configured to:
compute a score (170) for each procedure based on the computed values of the data quality metric in the procedure; and
display the score for one or more of the procedures; and/or
label the intraluminal device data for the procedure with the score; and/or
store to a computer readable storage medium, only the intraluminal device data for the procedure with the highest score.
